# EUROPÄISCHE PATENTSCHRIFT

(11) **EP 1 128 831 B1**
(45) Veröffentlichungstag und Bekanntmachung des Hinweises auf die Patenterteilung: **13.10.2004**
(21) Anmeldenummer: 99955906.5
(22) Anmeldetag: 29.10.1999
(51) Int. Cl.: A61K 31/4709, A61K 9/20, A61K 9/28, A61P 31/04

(54) **PHARMAZEUTISCHE ZUBEREITUNG VON MOXIFLOXACIN**
PHARMACEUTICAL MOXIFLOXACIN PREPARATION
PREPARATION PHARMACEUTIQUE DE MOXIFLOXACINE

(30) Priorität: 10.11.1998 DE 19855758
(43) Veröffentlichungstag der Anmeldung: 05.09.2001
(73) Patentinhaber: Bayer HealthCare AG, 51368 Leverkusen (DE)
(72) Erfinder: BOSCHE, Patrick, D-51519 Odenthal (DE); MAHLER, Hans, Friedrich, D-51061 Köln (DE); WEISEMANN, Claus, Apex, NC 27502 (US)
(86) Internationale Anmeldenummer: PCT/EP1999/008230
(87) Internationale Veröffentlichungsnummer: WO 2000/027398

(56) Entgegenhaltungen:
- EP-A- 0 350 733
- DE-A- 19 546 249

## Beschreibung

Die vorliegende Erfindung betrifft eine pharmazeutische Zubereitung zur oralen Anwendung, die Moxifloxacin, dessen Salz und/oder Hydrat sowie Lactose enthält, ein Verfahren zu ihrer Herstellung, sowie die Verwendung dieser Zubereitung zur Bekämpfung von bakteriellen Infektionen bei Menschen oder Tieren.

Moxifloxacin (INN - International Nonproprietary Name) ist ein Antibiotikum der folgenden Formel:

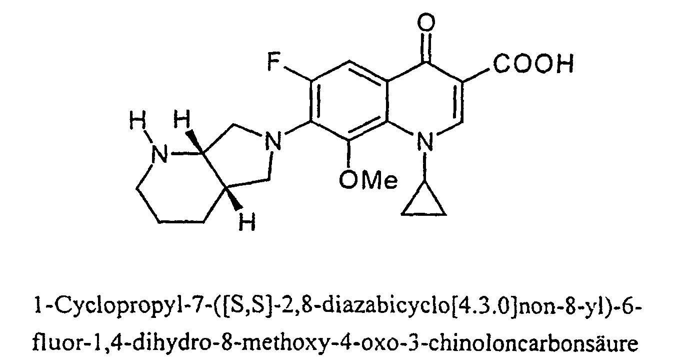

Es ist ein hochwirksames antiinfektives Mittel und wurde erstmals beschrieben in der EP-A-0 350 733. Die EP-A-0 350 733 beschreibt eine pharmazeutische Zubereitung, die den Wirkstoff, mikrokristalline Cellulose, Maisstärke, Poly-(1-vinyl)-2-pyrrolidon (unlöslich), hochdisperses Siliciumdioxid und Magnesiumstearat umfaßt.

Desweiteren sind in der EP-A-0 230 881 pharmazeutische Zubereitungen des Ciprofloxacins beschrieben, bei dem es sich ebenfalls um ein Antibiotikum aus der Klasse der Chinoloncarbonsäuren handelt. Die dort beschriebene Formulierung des Ciprofloxacins entspricht der in der EP-A-350 733 beschriebenen Formulierung. Wendet man diese im Stand der Technik bekannte Formulierung jedoch auf Moxifloxacin an, so zeigt sich unerwartet, daß aus ihr gefertigte Tabletten eine Härte bzw.

Bruchlast aufweisen, die in einigen Fällen beispielsweise bei Tablettenformulierungen für Blisterverpackungen verbesserungsfähig ist und auch hinsichtlich ihrer Freisetzungseigenschaften noch Raum für Verbesserungen bietet. Die Erfinder dieser Anmeldung stellten sich daher die Aufgabe, eine pharmazeutische Formulierung bereitzustellen, aus denen Tabletten mit ausreichender Härte bzw. Bruchlast hergestellt werden können, und die zur selben Zeit über ausgezeichnete Freisetzungseigenschaften verfügen.

Die Erfinder fanden überraschend, daß die zuvor beschriebene Aufgabenstellung gelöst werden kann durch eine pharmazeutische Formulierung, die Lactose in einem bestimmten Mengenanteil enthält.

Gegenstand der vorliegenden Erfindung sind somit pharmazeutische Zubereitungen zur oralen Anwendung, die Moxifloxacin, mindestens ein Trockenbindemittel, mindestens ein Zerfallhilfsmittel, und wahlweise ein Schmiermittel enthalten, dadurch gekennzeichnet, daß die Zubereitung 2,5 % bis 25% Lactose enthält (Alle %-Angaben sind Gewichtsprozente bezogen auf das Gewicht der pharmazeutischen Zubereitungen).

Desweiteren ist ein Verfahren zur Herstellung von Tabletten, die solche Zubereitungen enthalten, Gegenstand dieser Erfindung.

Bei der erfindungsgemäßen pharmazeutischen Zubereitung handelt es sich um eine Zubereitung zur oralen Anwendung.

Salze des Moxifloxacins schließen beispielsweise Säureadditionssalze, wie Salze von Salzsäure, Schwefelsäure, Essigsäure, Milchsäure etc. sowie Salze mit Basen, wie Natriumhydroxid, Kaliumhydroxid etc. und/oder Hydrate davon ein wie z.B. das erfindungsgemäß besonders bevorzugte Moxifloxacin-Hydrochlorid bzw. ein Monohydrat davon.

Die erfindungsgemäße Formulierung umfaßt bevorzugt von 50 bis 85 % besonders bevorzugt 60 bis 80 % Moxifloxacin oder Salze und/oder Hydrate davon.

Die pharmazeutische Zubereitung enthält bezogen auf die Einzeldosis im allgemeinen von 50 bis 800 mg Moxifloxacin, bevorzugt von 100 bis 600 mg, besonders bevorzugt von 200 bis 400 mg (jeweils bezogen auf die Betainform).

Die Anwendung der Lactose im erfindungsgemäßen Mengenbereich führt überraschend dazu, daß die Tablette, die aus der erfindungsgemäß pharmazeutischen Zubereitung hergestellt wird, über eine hervorragende Härte und Bruchlast und gleichzeitig über ausgezeichnete Freisetzungseigenschaften verfügt. Ein weiterer Vorteil der pharmazeutischen Zubereitung der vorliegenden Erfindung besteht darin, daß sie in einfacher Weise durch Granulation zugänglich ist, wobei man sowohl mikronisierten als auch nicht-mikronisierten Wirkstoff verwenden kann und in beiden Fällen zu bioäquivalenten Formulierungen gelangt.

Als zur Erreichung der erfindungsgemäßen Aufgabenstellung wesentlichen Bestandteil enthält die pharmazeutische Zubereitung 2,5 bis 25 % Lactose, bevorzugt von 5 bis 20 % Lactose und besonders bevorzugt von 7,5 bis 16 % Lactose. Erfindungsgemäß werden hier übliche Lactose-Monohydrat-Typen bevorzugt.

Die erfindungsgemäße pharmazeutische Zubereitung enthält mindestens ein Trockenbindemittel, das beispielsweise ausgewählt wird aus der Gruppe die besteht aus: mikrokristalliner Cellulose, Fasercellulose, Calciumphosphaten und Mannit. Besonders bevorzugt wird erfindungsgemäß mikrokristalline Cellulose verwendet. Diese ist im Handel beispielsweise unter der Bezeichnung Avicel® erhältlich. Die pharmazeutische Zubereitung enthält zweckmäßig 5 bis 30 %, bevorzugt 6,9 bis 30 %, besonders bevorzugt 12 bis 25 % des Trockenbindemittels.

Die erfindungsgemäße pharmazeutische Zubereitung enthält mindestens ein Zerfallhilfsmittel, das beispielsweise ausgewählt wird aus der Gruppe, die besteht aus Stärke, vorverkleisterte Stärke, Stärkeglykolate, quervernetztes Polyvinylpyrrolidon und Natriumcarboxymethylcellulose (= Croscarmellose-Natrium). Erfindungsgemäß ist besonders die Verwendung von Croscarmellose-Natrium bevorzugt. Die pharmazeutische Zubereitung enthält zweckmäßig 1 bis 10 %, bevorzugt 1,5 bis 8 %, besonders bevorzugt 2 bis 6 % des Zerfallhilfsmittels.

Die pharmazeutische Zubereitung der Erfindung enthält mindestens ein Schmiermittel, das ausgewählt wird aus der Gruppe der Fettsäuren und deren Salze. Erfindungsgemäß besonders bevorzugt ist die Verwendung von Magnesiumstearat. Das Schmiermittel wird zweckmäßig in einer Menge von 0,3 bis 2,0 %, besonders bevorzugt von 0,4 bis 1,5 % und am meisten bevorzugt von 0,5 bis 1,1 % eingesetzt.

Eine besonders bevorzugte pharmazeutische Zubereitung der Erfindung enthält:
von 60 bis 70 % Moxifloxacin oder Salze und/oder Hydrate davon,
von 7,5 bis 16 % Lactose,
von 2 bis 6 % Croscarmellose-Natrium,
von 0,5 bis 1,1 % Magnesiumstearat sowie
bis zu 30 % mikrokristalline Cellulose.

Die pharmazeutische Zubereitung der Erfindung kann zweckmäßig durch ein Verfahren hergestellt werden, bei dem Moxifloxacin, dessen Salz und/oder Hydrat, mindestens ein Trockenbindemittel und Lactose wäßrig granuliert werden, das Granulat anschließend mit mindestens einem Zerfallhilfsmittel und mindestens einem Schmiermittel vermischt und gegebenenfalls tablettiert und lackiert wird.

Zur Granulation werden Verfahren nach dem Prinzip der Schnellmischergranulation verwendet. Die Granulation kann mit Wasser ohne Zusatz eines Klebemittels erfolgen.

Die pharmazeutische Zubereitung der vorliegenden Erfindung wird besonders bevorzugt in Form einer Tablettenformulierung angewendet, die gegebenenfalls lackiert sein kann (Wie bereits oben erwähnt, beziehen sich die Gewichtsangaben in der vorliegenden Patentanmeldung auf das Gesamtgewicht der pharmazeutischen Zubereitung ohne das Gewicht der wahlweise vorhandenen Lackierung). Zur Lackierung können in der pharmazeutischen Technologie übliche Lackierungsformulierungen angewendet werden, wie z.B. solche auf Basis von Hydroxypropylmethylcellulose (HPMC) und/oder Polyethylenglykol verschiedener Molekulargewichte. Weiterhin kann die Lackierung übliche Pigmente enthalten, wie z. B. Titandioxid oder rotes Eisenoxid.

Die erfindungsgemäße pharmazeutische Zubereitung wird bevorzugt zur Behandlung oder Prävention von bakteriellen Infektionen bei Menschen oder Tieren verwendet.

### Beispiele

### Beispiel 1

Tablette enthaltend 50 mg Moxifloxacin als mikronisierter Wirkstoff, Wirkstoffgehalt ca. 66% (bezogen auf unlackierte Tablette):

| | |
|---|---|
| Moxifloxacin Hydrochlorid, mikronisiert | 54,6 mg |
| Mikrokristalline Cellulose | 17,0 mg |
| Lactose | 8,5 mg |
| Croscarmellose Natrium | 2,0 mg |
| Magnesiumstearat | 0,6 mg |
| HPMC Lack | 3,2 mg |

### Beispiel 2

Tablette enthaltend 50 mg Moxifloxacin als mikronisierter Wirkstoff, Wirkstoffgehalt ca. 80% (bezogen auf unlackierte Tablette)

| | |
|---|---|
| Moxifloxacin Hydrochlorid, mikronisiert | 54,6 mg |
| Mikrokristalline Cellulose | 7,1 mg |
| Lactose | 3,55 mg |
| Croscarmellose Natrium | 2,7 mg |
| Magnesiumstearat | 0,4 mg |

### Beispiel 3

Tablette enthaltend 50 mg Moxifloxacin als mikronisierter Wirkstoff, Wirkstoffgehalt ca. 65% (bezogen auf unlackierte Tablette)

| | |
|---|---|
| Moxifloxacin Hydrochlorid, mikronisiert | 54,6 mg |
| Mikrokristalline Cellulose | 12,8 mg |
| Lactose | 12,8 mg |
| Croscarmellose Natrium | 3,4 mg |
| Magnesiumstearat | 0,5 mg |

### Beispiel 4

Tablette enthaltend 200 mg Moxifloxacin als mikronisierter Wirkstoff:

| | |
|---|---|
| Moxifloxacin Hydrochlorid, mikronisiert | 218,4 mg |
| Mikrokristalline Cellulose | 68,0 mg |
| Lactose | 34,0 mg |
| Croscarmellose Natrium | 8,0 mg |
| Magnesiumstearat | 2,4 mg |
| HPMC Lack | 9,0 mg |

### Beispiel 5

Tablette enthaltend 400 mg Moxifloxacin als mikronisierter Wirkstoff:

| | |
|---|---|
| Moxifloxacin Hydrochlorid, mikronisiert | 436,8 mg |
| Mikrokristalline Cellulose | 136,0 mg |
| Lactose | 68,0 mg |
| Croscarmellose Natrium | 16,0 mg |
| Magnesiumstearat | 4,8 mg |
| HPMC Lack | 14,0 mg |

### Beispiel 6

Tablette enthaltend 400 mg Moxifloxacin als nicht-mikronisierter Wirkstoff:

| | |
|---|---|
| Moxifloxacin Hydrochlorid | 436,8 mg |
| Mikrokristalline Cellulose | 136,0 mg |
| Lactose | 68,0 mg |
| Croscarmellose Natrium | 32,0 mg |
| Magnesiumstearat | 6,0 mg |
| HPMC Lack | 21,0 mg |

**Abbildung 1** zeigt den Vergleich der Bruchfestigkeit von Tabletten gemäß Beispiel 6 und der Formulierung gemäß EP-A-0 350 733 (Seite 53).

**Abbildung 2** zeigt den Vergleich der Freisetzung von Moxifloxacin HCl aus Tabletten gemäß Beispiel 6 und der Formulierung nach EP-A-0 350 733.

Die Herstellung der Tabletten erfolgte jeweils im Labormaßstab unter vergleichbaren Granulations- und Tablettierbedingungen.

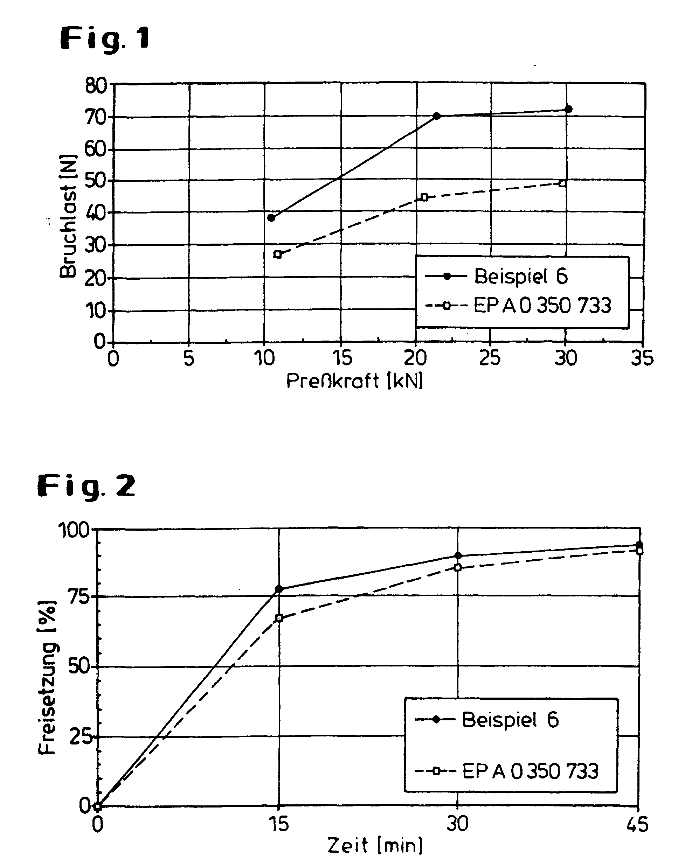

Abbildung 1 zeigt deutlich die verbesserte Tablettenhärte der erfindungsgemäßen Formulierung. Trotzdem ergibt sich eine schnellere Wirkstoffliberation, wie Abbildung 2 zeigt.

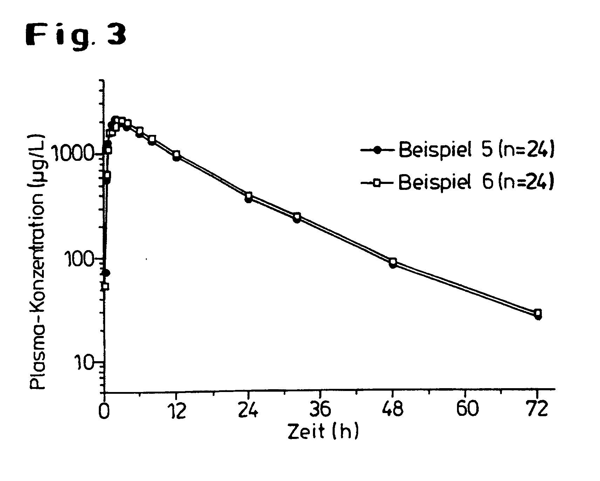

**Abbildung 3** zeigt den Vergleich der Blutspiegelkurven von Tabletten gemäß Beispiel 5 und Beispiel 6.

Vergleicht man Tabletten gemäß Beispiel 5 (mikronisiertes Moxifloxacin HCl) mit Tabletten gemäß Beispiel 6 (nicht-mikronisiertes Moxifloxacin HCl) bezüglich ihrer Bioäquivalenz, zeigt sich ein weiterer unerwarteter Vorteil der erfindungsgemäßen Formulierung darin, daß trotz der deutlich unterschiedlichen Wirkstoffpartikelgrößen beide Formulierungen zu gleichen Blutspiegelverläufen führen. Dadurch ist bei der Formulierung der Erfindung ein Mikronisierungsschritt entbehrlich, was zu Kostenvorteilen führt.

## Patentansprüche

1. Pharmazeutische Zubereitung zur oralen Anwendung, die
Moxifloxacin oder ein Salz und/oder Hydrat davon,
mindestens ein Trockenbindemittel,
mindestens ein Zerfallhilfsmittel, und
mindestens ein Schmiermittel enthält,
**dadurch gekennzeichnet, daß** die Zubereitung 2,5 % bis 25 % Lactose enthält.

2. Pharmazeutische Zubereitung zur oralen Anwendung nach Anspruch 1, **dadurch gekennzeichnet, daß** die Zubereitung von 50 bis 800 mg Moxifloxacin oder dessen Salze und/oder Hydrate bezogen auf eine Einzeldosierung enthält.

3. Pharmazeutische Zubereitung zur oralen Anwendung nach Anspruch 1 oder 2, **dadurch gekennzeichnet, daß** das Trockenbindemittel mikrokristalline Cellulose ist.

4. Pharmazeutische Zubereitung zur oralen Anwendung nach einem der Ansprüche 1 bis 3, **dadurch gekennzeichnet, daß** das Zerfallhilfsmittel Croscarmellose-Natrium ist.

5. Pharmazeutische Zubereitung zur oralen Anwendung nach irgendeinem der Ansprüche 1 bis 4, **dadurch gekennzeichnet, daß** das Schmiermittel Magnesiumstearat ist.

6. Pharmazeutische Zubereitung zur oralen Anwendung nach irgendeinem der Ansprüche 1 bis 5, **dadurch gekennzeichnet, daß** sie enthält:
von 60 bis 76 % Moxifloxacin oder ein Salz und/oder Hydrat davon,
von 7,5 bis 16 % Lactose,
von 2 bis 6 % Croscarmellose-Natrium,
von 0,5 bis 1,1 % Magnesiumstearat,
und bis zu 30 % mikrokristalline Cellulose.

7. Tablettenzubereitung zur oralen Anwendung, die einen Kern aus der pharmazeutischen Zubereitung nach irgendeinem der Ansprüche 1 bis 6 und einen Lacküberzug umfaßt.

8. Pharmazeutische Zubereitung zur oralen Anwendung nach irgendeinem der Ansprüche 1 bis 7, **dadurch gekennzeichnet, daß** sie Moxifloxacin-Hydrochlorid enthält.

9. Pharmazeutische Zubereitung zur oralen Anwendung nach irgendeinem der Ansprüche 1 bis 8, zur Bekämpfung von bakteriellen Erkrankungen bei Menschen oder Tieren.

10. Verfahren zur Herstellung der pharmazeutischen Zubereitung zur oralen Anwendung nach irgendeinem der Ansprüche 1 bis 8, **dadurch gekennzeichnet, daß** Moxifloxacin, dessen Salz und/oder Hydrat, mindestens ein Trockenbindemittel und Lactose wäßrig granuliert werden, das Granulat anschließend mit mindestens einem Zerfallhilfsmittel und mindestens einem Schmiermittel vermischt und gegebenenfalls tablettiert und lackiert.

## Claims

1. Pharmaceutical preparation for oral administration which comprises
moxifloxacin or a salt and/or hydrate thereof,
at least one dry binder,
at least one disintegrant, and
at least one lubricant,
**characterized in that** the preparation comprises from 2.5% to 25% of lactose.

2. Pharmaceutical preparation for oral administration according to Claim 1, **characterized in that** the preparation comprises from 50 to 800 mg of moxifloxacin or its salts and/or hydrates, based on an individual dosage.

3. Pharmaceutical preparation for oral administration according to Claim 1 or 2, **characterized in that** the dry binder is microcrystalline cellulose.

4. Pharmaceutical preparation for oral administration according to any of Claims 1 to 3, **characterized in that** the disintegrant is croscarmellose sodium.

5. Pharmaceutical preparation for oral administration according to any of Claims 1 to 4, **characterized in that** the lubricant is magnesium stearate.

6. Pharmaceutical preparation for oral administration according to any of Claims 1 to 5, **characterized in that** it comprises:
from 60 to 76% of moxifloxacin or a salt and/or hydrate thereof,
from 7.5 to 16% of lactose,
from 2 to 6% of croscarmellose sodium,
from 0.5 to 1.1 % of magnesium stearate,
and up to 30% of microcrystalline cellulose.

7. Tablet preparation for oral administration which comprises a core of the pharmaceutical preparation according to any of Claims 1 to 6 and a film coating.

8. Pharmaceutical preparation for oral administration according to any of Claims 1 to 7, **characterized in that** it comprises moxifloxacin hydrochloride.

9. Pharmaceutical preparation for oral administration according to any of Claims 1 to 8, for controlling bacterial infections in humans or animals.

10. Process for preparing the pharmaceutical preparation for oral administration according to any of Claims 1 to 8, **characterized in that** moxifloxacin, its salt and/or hydrate, at least one dry binder and lactose are granulated using water, the granules are subsequently mixed with at least one disintegrant and at least one lubricant and, if appropriate, tabletted and coated.

## Revendications

1. Préparation pharmaceutique à administrer par voie orale, qui contient
de la moxifloxacine ou un sel et/ou hydrate de moxifloxacine,
au moins un liant sec,
au moins un auxiliaire de désintégration, et
au moins un agent lubrifiant,
**caractérisée en ce qu'**elle contient 2,5 à 25 % de lactose.

2. Préparation pharmaceutique à administrer par voie orale suivant la revendication 1, **caractérisée en ce qu'**elle contient une quantité, rapportée à une dose individuelle, de 50 à 800 mg de moxifloxacine ou de ses sels et/ou hydrates.

3. Préparation pharmaceutique à administrer par voie orale suivant la revendication 1 ou 2, **caractérisée en ce que** le liant sec est de la cellulose microcristalline.

4. Préparation pharmaceutique à administrer par voie orale suivant l'une des revendications 1 à 3, **caractérisée en ce que** l'auxiliaire de désintégration est du croscarmellose sodique.

5. Préparation pharmaceutique à administrer par voie orale suivant l'une quelconque des revendications 1 à 4, **caractérisée en ce que** le lubrifiant est du stéarate de magnésium.

6. Préparation pharmaceutique à administrer par voie orale suivant l'une quelconque des revendications 1 à 5, **caractérisée en ce qu'**elle contient :
de 60 à 76 % de moxifloxacine ou d'un sel et/ou d'un hydrate de moxifloxacine,
de 7,5 à 16 % de lactose,
de 2 à 6 % de croscarmellose sodique,
de 0,5 à 1,1 % de stéarate de magnésium,
et jusqu'à 30 % de cellulose microcristalline.

7. Préparation en comprimés à administrer par voie orale, qui comprend un noyau formé de la préparation pharmaceutique suivant l'une quelconque des revendications 1 à 6 et un revêtement de laque.

8. Préparation pharmaceutique à administrer par voie orale suivant l'une quelconque des revendications 1 à 7, **caractérisée en ce qu'**elle contient du chlorhydrate de moxifloxacine.

9. Préparation pharmaceutique à administrer par voie orale suivant l'une quelconque des revendications 1 à 8, destinée à combattre des maladies bactériennes chez l'homme ou l'animal.

10. Procédé de production de la préparation pharmaceutique à administrer par voie orale suivant l'une quelconque des revendications 1 à 8, **caractérisée en ce que** de la moxifloxacine, un sel et/ou un hydrate de moxifloxacine, au moins un liant sec et du lactose sont granulés par voie aqueuse, les granules sont ensuite mélangés avec au moins un auxiliaire de désintégration et au moins un lubrifiant et le mélange est éventuellement transformé en comprimés et enrobé de laque.
